# EUROPEAN PATENT APPLICATION

(11) **EP 2 543 351 A1**
(43) Date of publication of application: **09.01.2013**
(21) Application number: 11750470.4
(22) Date of filing: 09.02.2011
(51) Int. Cl.: A61F 13/15, A61F 13/472, B29C 59/04, B31F 1/07

(54) **ROTARY PROCESSING DEVICE**

(30) Priority: 01.03.2010 JP 2010044372
(71) Applicant: Unicharm Corporation, Ehime 799-0111 (JP)
(72) Inventor: OBA, Kenji, Kanonji-shi Kagawa 769-1602 (JP)
(74) Representative: Peter, Julian
(86) International application number: PCT/JP2011/053306
(87) International publication number: WO 2011/108357

(57) **Abstract**

A rotary processing device (20) comprises a plurality of processing rolls (RP1, RP2), an anvil roll (RA) arranged facing the processing rolls, and an upstream nip roll (RNU) and a downstream nip roll (RND) arranged on the upstream and downstream of the processing rolls while facing the anvil roll. A belt like body (W) is so carried as to pass through between the upstream nip roll and the anvil roll and, next, through between the processing roll and the anvil roll and, thereafter, through between downstream nip roll and the anvil roll, so as to be processed by the processing rolls.

## Description

### Technical Field

This invention relates to a rotary processing device.

### Background Art

A rotary processing device which executes two kinds of embossings for a belt-like body that is carried, comprising two embossing rolls and a single anvil roll arranged facing the embossing rolls, the belt like body being carried so as to successively pass through between the embossing rolls and the anvil roll and as to be embossed by the two embossing rolls (see PLT 1) is known. In this rotary processing device, a convex portion formed on one embossing roll concavely embosses the belt like body, and a protrusion formed on the other embossing roll perforates the bottom of the concave embossment.

In this case, the concave embossment and the protrusion must be correctly positioned in the direction of carry. According to PLT 1, the convex portion formed on one embossing roll and the concave portion formed in the anvil roll are brought in mesh together to concavely emboss the belt like body, and the concave embossment is carried while being held in the concave portion. Next, the protrusion formed on the other embossing roll and the concave portion of the anvil roll are brought in mesh together to perforate the bottom of the concave embossment.

### Citation List

### Patent Literature

PLT 1: Japanese Unexamined Patent Publication No. 2008-18537)

### Summary of Invention:

### Technical Problem

However, considering that it is common to carry the belt like body while applying a tensile force to the belt like body in the direction of carry, it is difficult to carry the belt-like body with the concave embossment being held in the concave portion of the anvil roll. Thus, the concave embossment and the protrusion may not be brought into the correct position in the direction of carry.

PLT 1 is presuming, as a prerequisite, a special case where the anvil roll has concave portions in the circumferential surface thereof. Therefore, the art of PLT 1 cannot be applied to a general case where the circumferential surface of the anvil roll is assuming a cylindrical surface.

### Solution to Problem

According to the present invention, there is provided a rotary processing device which executes a plurality of processings for a belt-like body that is carried, comprising a plurality of processing rolls; a single anvil roll arranged facing the processing rolls; and an upstream nip roll and a downstream nip roll arranged on the upstream and downstream of the processing rolls while facing the anvil roll; wherein the belt-like body is so carried as to pass through between the upstream nip roll and the anvil roll and, next, through between the processing roll and the anvil roll and, thereafter, through between the downstream nip roll and the anvil roll, so as to be processed by the processing rolls.

### Advantageous Effects of Invention

A plurality of processings is executed for the belt-like body being correctly positioned.

### Brief Description of the Drawings

Fig. 1 is a front view of an absorbent article;
Fig. 2 is a schematic transverse sectional view of the absorbent article as viewed along the line II-II in Fig. 1;
Fig. 3 is a front view of the absorbent article of when flaps are developed;
Fig. 4 is a schematic view of a process for producing the absorbent article;
Fig. 5 is a schematic front view of a rotary processing device;
Fig. 6 is a schematic back view of the rotary processing device; and
Fig. 7 is a view schematically illustrating the steps of processing in the rotary processing device.

### Description of Embodiments

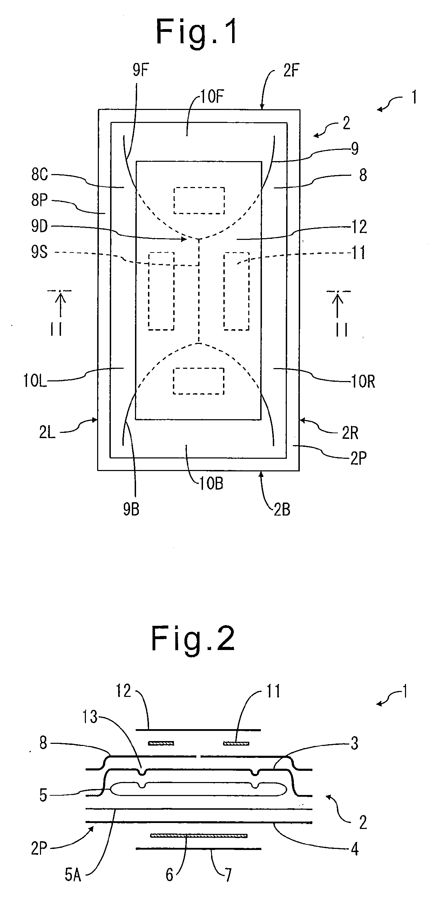

Referring to Figs. 1 to 3, an absorbent article 1 such as sanitary napkin, panty liner or incontinence pad includes an absorptive body 2. The absorptive body 2 includes a fluid-permeable top sheet 3, a fluid-impermeable back sheet 4, and a fluid-retaining absorptive body 5 arranged between the top sheet 3 and the back sheet 4. The top sheet 3 and the back sheet 4 are substantially of the same size, and are joined and sealed together along the circumferential edge portion 2P thereof by a heat-seal processing or by using a hot-melt adhesive. Further, a sticking agent 6 is applied onto the outer surface of the back sheet 4 which when in use faces the clothing such as underwear so that the absorptive body 2 can be stuck to the underwear. The sticking agent 6 has been covered with a removable sheet piece 7.

Note that, in Fig. 1, reference numerals 2F, 2B, 2L and 2R denote front edge, rear edge, left edge and right edge of the napkin 1 or the absorptive body 2, respectively. The front, rear, right and left in this case correspond to the front, rear, right and left of the body of a wearer. In addition, in Fig. 2, reference numeral 5A denotes a tissue arranged between the absorptive body 5 and the back sheet 3.

The surface of the top sheet 3 which when in use comes in contact with the wearer, has been covered with a fluid-impermeable cover sheet 8. The cover sheet 8 has substantially the same size as the absorptive body 2 and is, therefore, covering almost the whole surface of the top sheet 3. The cover sheet 8 is, further, joined to the top sheet 3 or the absorptive body 2 along an annular circumferential edge region 8P thereof by a heat-seal processing or by using a hot-melt adhesive. In contrast with this, a central region 8C which is the region other than the circumferential edge region 8P has not been joined to the top sheet 3.

A slit 9 has been formed in advance in the central region 8C of the cover sheet 8. Due to the slit 9, a plurality of flaps are defined or formed on the central region 8C for sticking the absorptive body 2 to the underwear.

In this connection, the slit 9 is formed of a cutting line for completely cutting the cover sheet 8. Alternatively, the slit 9 may be formed of a weakened line, such as perforations.

In an example shown in Fig. 1, the slit 9 is constituted by a U-shaped curved portion 9F that opens wide toward the front edge 2F, a U-shaped curved portion 9B that opens wide toward the rear edge 2B, and a straight portion 9S which connects the vertexes of the curved portions 9F and 9B together substantially at the center of the cover sheet 8. As a result, four flaps are defined on the central region 8C, i.e., half elliptic front flap 10F and rear flap 10B, and trapezoidal left flap 10L and right flap 10R. In this case, the front and rear flaps 10F and 10B are positioned symmetrically to each other, and the right and left flaps 10R and 10L, too, are positioned symmetrically to each other.

In this connection, at the points where the curved portions 9F and 9B are connected to the straight line 9S, the slit 9 may be branched into three. Namely, the slit 9 includes branching portions 9D.

An adhesive 11 has been applied to the outer surfaces of the flaps 10F, 10B, 10L and 10R so that the corresponding flaps 10F, 10B, 10L and 10R can be stuck to the underwear. The adhesive 11 has been covered with a common removable sheet 12.

In this case, the removable sheet piece 12 is provided spanning across the slit 9 and, specifically, covering the branching portions 9D. This prevents the flaps 10F, 10B, 10L and 10R from being stripped off or wrinkled.

Materials of the elements will be described below.

The top sheet 3 is constituted by, for example, a porous or non-porous non-woven fabric or a porous plastic sheet.

The back sheet 4 is constituted by, for example, a hydrophobic non-woven fabric, a water-impermeable plastic film, a laminated sheet of a non-woven fabric and a water-impermeable plastic film, a highly water-resistant melt-blown non-woven fabric or an SMS non-woven fabric sandwiched by a highly strong spun-bonded non-woven fabric.

The absorptive body 5 is constituted by, for example, a fluff-like pulp or an air-laid non-woven fabric and a highly absorptive polymer. In this connection, the flap-like pulp is constituted by an artificial cellulose fiber such as chemical pulp, cellulose fiber, rayon or acetate, the air-laid non-woven fabric is constituted by, for example, a non-woven fabric obtained by heat-melt-adhering a pulp and a synthetic fiber together and sticking them with a binder, and the highly absorptive polymer is constituted by, for example, a particulate or fibrous polymer of the type of starch, acrylic acid or amino acid.

The sticking agents 6 and 11 are constituted by a hot-melt adhesive, such as styrene/isoprene/styrene block copolymer (SIS), styrene/butadiene/styrene block copolymer (SBS) or styrene/ethylene/butylene/ethylene copolymer (SEBS).

The cover sheet 8 is constituted by, for example, a hydrophobic non-woven fabric, a water-impermeable plastic film, a laminated sheet of a non-woven fabric and a water-impermeable plastic film, a highly water-resistant melt-blown non-woven fabric or an SMS non-woven fabric sandwiched by a highly strong spun-bonded non-woven fabric and is, preferably, constituted by the hydrophobic non-woven fabric. The cover sheet 8 has a basis weight of, desirably, from 15 g/m² to 60 g/m². When the cover sheet 8 is constituted by a plastic film, it is desired that the draping of the plastic film is 20 mm to 100 mm and, preferably, 30 mm to 70 mm as measured by the cantilever method. If the draping is less than 20 mm, the cover sheet 8 tends to be twisted. If the draping exceeds 100 mm, on the other hand, the cover sheet 8 becomes so hard that the feeling of use of the napkin 1 may be deteriorated and that the napkin 1 follows up the underwear less which may cause the absorbed fluid to leak.

The above-mentioned cantilever method complies with the JIS-L1018 and is conducted as described below. Namely, five pieces of an object to be measured 150 mm long and 25 mm wide are overlapped to obtain a sample for measurement. Next, by using a cantilever manufactured by Daiei Kagaku Seiki Mfg. Co., the sample to be measured is held under a holder plate of the cantilever, and is caused to slide in the direction of a tilted surface at a speed of 5 mm/sec to automatically measure the distance of travel. Measurements are taken for both of when the front surface of the sample to be measured is facing downward and when the back surface of the sample to be measured is facing downward. The measurements are averaged and are regarded to be the measured result.

The absorbent article 1 is stuck to the underwear as described below. First, the removable sheet piece 7 on the side of the back sheet 4 is removed, and the absorbent article 1 or the absorptive body 2 is stuck to the underwear via the sticking agent 6. At this time, the top sheet 3 has been covered with the cover sheet 8 and is prevented from being fouled.

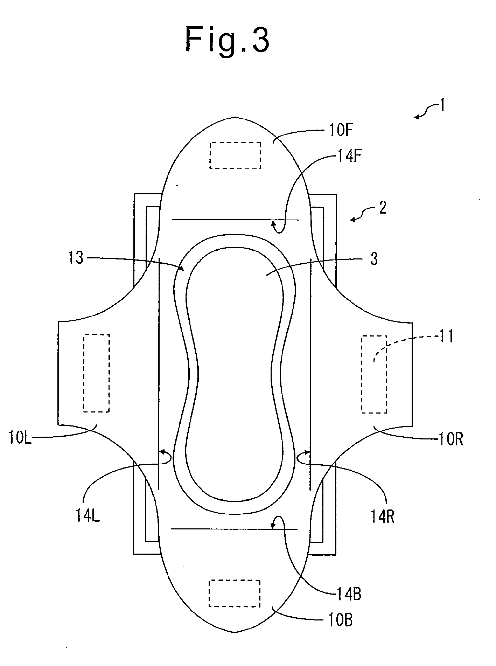

Next, as shown in Fig. 3, the flaps 10F, 10B, 10L and 10R are developed. Namely, the front flap 10F is folded at a folding region 14F along the front edge 2F to be developed forward, and the rear flap 10B is folded at a folding region 14B along the rear edge 2B to be developed rearward. Further, the left flap 10L is folded at a folding region 14L along the left edge 2L to be developed leftward, and the right flap 10RL is folded at a folding region 14RL along the right edge 2RL to be developed rightward. As a result, the top sheet 3 is exposed.

Next, the flaps 10F, 10B, 10L and 10R are stuck to the underwear via the sticking agent 11. Namely, the front flap 10F and the rear flap 10B are stuck to the inner surface of the underwear, while the left flap 10L and the right flap 10R are stuck to the outer surface of the underwear on the lower side of the absorbent article 1.

As a result, despite external forces act on the absorptive body 2 from various directions, the absorptive body 2 is suppressed from being twisted or deviated in position. Namely, the absorptive body 2 can be reliably stuck to the underwear and, hence, the absorbed fluid can be suppressed from leaking. Further, the wearer does not have to worry about the leakage.

Referring to Figs. 2 and 3, further, a leakage-preventing groove 13 has been formed in the absorptive body 2 on the side of the person who wears it. This further helps suppress the leakage.

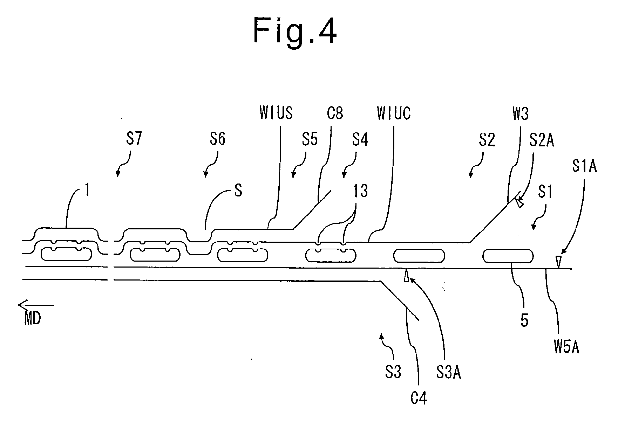

Fig. 4 schematically shows a process for producing the absorbent articles 1.

Referring to Fig. 4, a tissue web W5A which is a continuous body of tissues 5A is carried in a direction MD of carry, and absorptive bodies 5 are intermittently arranged on the tissue web W5A, i.e., arranged maintaining a gap in the direction MD of carry (S1). An adhesive such as hot-melt adhesive has been applied in advance on the upper surface of the tissue web W5A (S1A), and the absorptive bodies 5 are fixed onto the tissue web W5A due to the adhesive. Next, a top sheet web W3 which is a continuous body of the top sheets 3 is overlapped on the absorptive bodies 5 (S2). An adhesive has been applied in advance on the inner surface of the top sheet web W3, i.e., on the surface facing the absorptive bodies 5 (S2A), and the top sheet web W3 is stuck to the absorptive bodies 5 due to the adhesive.

Next, a back sheet composite body C4 is overlapped onto the lower surface of the tissue web W5A to thereby form an uncovered intermediate product web WIUC (S3). The back sheet composite body C4 comprises a back sheet web which is a continuous body of the back sheets 4 on which the sticking agent 6 and the removable sheet pieces 7 are intermittently applied. An adhesive has been applied in advance on the bottom surface of the tissue web W5A, i.e., on the surface facing the back sheet composite body C4 (S3A), and the back sheet composite body C4 is stuck to the tissue web W5A due to the adhesive.

Next, the uncovered intermediate product web WIUC is intermittently embossed on the side of the top sheet web W3 to thereby intermittently form the leakage-preventing grooves 13 (S4).

Next, a cover sheet composite body C8 is overlapped on the uncovered intermediate product web WIUC on the side of the top sheet web W3 to thereby form an unsealed intermediate product web WIUS (S5). The cover sheet composite body C8 comprises a cover sheet web W8 which is a continuous body of the cover sheets 8 on which the sticking agent 11 and the removable sheet pieces 12 are intermittently applied.

Next, a sealed portion S is formed on the unsealed intermediate product web WIUS along the entire circumference of the absorptive bodies 5 to thereby form a sealed intermediate product web WIS (S6). The uncovered intermediate product web WIUC, unsealed intermediate product web WIUS and sealed intermediate product web WIS can be all regarded to be constituting an intermediate product web which is a continuous body of the intermediate products of the absorbent articles 1.

Next, the sealed portion S is cut (S7) along the direction at right angles with the direction MD of carry to thereby complete the absorbent article 1.

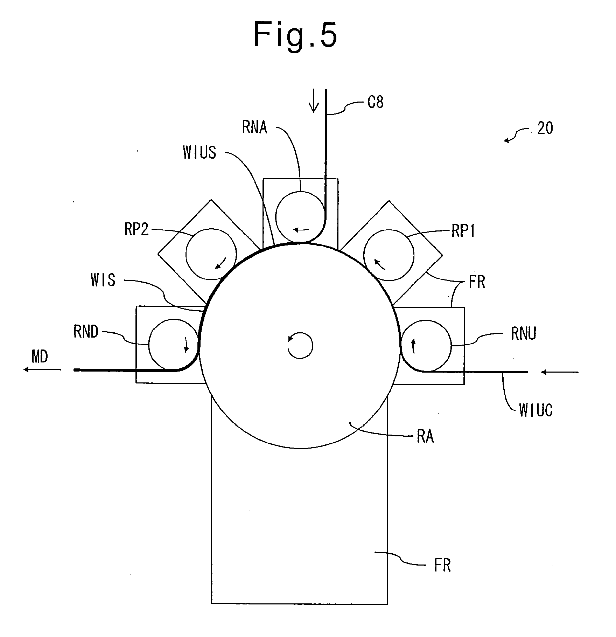

According to the embodiment of the invention, the step (S4) of forming the leakage-preventing groove 13, the step (S5) of overlapping the cover sheet composite body C8 and the step (S6) of forming the sealed portion S are executed by a rotary processing device 20 shown in Fig. 5.

Referring to Fig. 5, the rotary processing device 20 comprises a plurality of, for example, two processing rolls RP1 and RP2, a single anvil roll RA arranged facing the processing rolls RP1 and RP2, an upstream nip roll RNU arranged facing the anvil roll RA on the upstream of the processing rolls RP1 and RP2, a downstream nip roll RND arranged facing the anvil roll RA on the downstream of the processing rolls RP1 and RP2, and an additional nip roll RNA arranged facing the anvil roll RA between the upstream nip roll RNU and the downstream nip roll RND or, more correctly, between the processing rolls RP1 and RU2. The rolls RP1, RP2, RA, RNU, RND and RNA are rotatably supported by their corresponding frames FR in a manner that their axes of rotation extend in a horizontal direction in parallel with each other.

The processing rolls RP1 and RP2 are constituted by rolls which intermittently execute, for example, the embossing for the belt like body. Specifically, the processing roll RP1 presses the uncovered intermediate product web WIUC onto the anvil roll RA to intermittently form the leakage-preventing grooves 13, and the processing roll RP2 presses the unsealed intermediate product web WIUS onto the anvil roll RA to intermittently form the sealed portions S.

As can be seen from Fig. 5, further, the processing rolls RP1 and RP2 are arranged over the anvil roll RA. This reduces the load acting on the frames RF supporting the processing rolls RP1 and RP2.

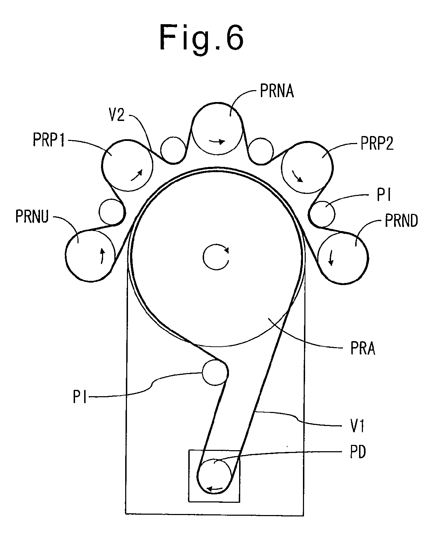

Further, the processing rolls RP1 and RP2, anvil roll RA, and nip rolls RNU, RND and RNA are rotated by the same driving machine. That is, as shown in Fig. 6, an endless belt V1 is wrapped round a pulley PD of the driving machine such as servo motor and round a pulley PRA of the anvil roll RA. Therefore, the anvil roll RA is rotated by the endless belt V1. Further, another endless belt V2 is wrapped round the pulley PRA, pulleys PRP1 and PRP2 of the processing rolls RP1 and RP2, and nip rolls RNU, RND and RNA. As the anvil roll RA rotates, therefore, the other rolls RP1, RP2, RA, RNU and RNA, are also rotated due to the endless belt V2.

In this case, the processing rolls RP1 and RP2, and nip rolls RNU, RND and RNA rotate in a direction opposite to the anvil roll RA. Note that, in Fig. 6, PI denotes an idler pulley.

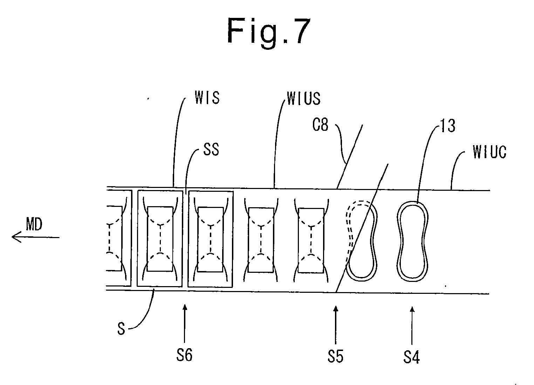

Referring to Figs. 5 and 7, the uncovered intermediate product web WIUC passes through between the upstream nip roll RNU and the anvil roll RA and, further, passes through between the processing roll RP1 and the anvil roll RA where the processing roll RP1 intermittently forms the leakage-preventing grooves 13 in the uncovered intermediate product web WIUC (S4). Next, the uncovered intermediate product web WIUC passes through between the additional nip roll RNA and the anvil roll RA.

On the other hand, the cover sheet composite body C8 is fed to between the additional nip roll RNA and the anvil roll RA, and is overlapped on the uncovered intermediate product web WIUC to thereby form the unsealed intermediate product web WIUS (S5).

The unsealed intermediate product web WIUS then passes through between the processing roll RP2 and the anvil roll RA where the processing roll RP2 forms the sealed portions S in the unsealed intermediate product web WIUS (S6). Note that the sealed portions SS of the sealed portions S that extend in the direction at right angles with the direction MD of carry may be formed by maintaining a gap in the direction MD of carry.

In this connection, the nip rolls RNU, RND and RNA process to carry the belt like bodies such as the intermediate product webs WIUC, WIUS and WIS while pressing them onto the anvil roll RA. Therefore, almost no tensile force acts on the belt like bodies in the direction MD of carry between the nip rolls neighboring to one another, i.e., almost no tensile force acts between the upstream nip roll RNU and the additional nip roll RNA, and between the additional nip roll RNA and the downstream nip roll RND. Note that this will be also understood from the fact that the nip rolls RNU, RND and RNA are rotating at a substantially equal speed.

As a result, the processing rolls RP1 and RP2 execute the processings for the belt like bodies in the state where almost no tensile force is acting thereon. Accordingly, the processing positions are correctly maintained, and the plurality of processings can be correctly positioned.

As described with reference to Fig. 6, further, the processing rolls RP1, RP2, the anvil roll RA, and the nip rolls RNU, RND and RNA are driven by the same driving machine. As a result, the rotations of these rolls can be easily brought into synchronism making it, therefore, it is possible to further suppress deviation among the processing positions and deviation among the positions of the members.

### Reference Signs List

- 1: absorbent article
- RP1, RP2: processing rolls
- RA: anvil roll
- RNU: upstream nip roll
- RND: downstream nip roll

## Claims

1. A rotary processing device which executes a plurality of processings for a belt like body that is carried, comprising:
a plurality of processing rolls;
a single anvil roll arranged facing the processing rolls; and
an upstream nip roll and a downstream nip roll arranged on the upstream and downstream of the processing rolls while facing the anvil roll; wherein
the belt like body is so carried as to pass through between the upstream nip roll and the anvil roll and, next, through between the processing roll and the anvil roll and, thereafter, through between downstream nip roll and the anvil roll, so as to be processed by the processing rolls.

2. The rotary processing device according to claim 1, wherein the processing rolls include a plurality of rolls that intermittently execute emboss processings for the belt like body.

3. The rotary processing device according to claim 1 or 2, wherein at least one additional nip roll is arranged facing the anvil roll between the upstream nip roll and the downstream nip roll.

4. The rotary processing device according to any one of claims 1 to 3, wherein the processing rolls, the anvil roll and the nip rolls are rotated by the same driving machine.

5. The rotary processing device according to any one of claims 1 to 4, wherein the axes of rotation of the processing rolls, the anvil roll and the nip rolls are extending in a horizontal direction, and the processing rolls are arranged over the anvil roll.

6. The rotary processing device according to any one of claims 1 to 5, wherein the belt like body is constituted by an intermediate product web which is a continuous body of intermediate products of the absorbent articles, and the processing rolls include a roll which intermittently forms leakage-preventing grooves in an absorptive body web and a roll which intermittently forms sealed portions in the absorptive body web.
